# EUROPEAN PATENT SPECIFICATION

(11) **EP 1 917 005 B1**
(45) Date of publication and mention of the grant of the patent: **08.09.2010**
(21) Application number: 06708239.6
(22) Date of filing: 13.02.2006
(51) Int. Cl.: A61K 9/70, A61K 9/50, A61K 33/00

(54) **DEVICE, SYSTEM, AND METHOD COMPRISING MICROENCAPSULATED PROTON DONOR FOR RELEASE OF NITRIC OXIDE FROM A POLYMER**
VORRICHTUNG UND VERFAHREN MIT EINEM MIKROVERKAPSELTEN PROTONENSPENDER ZUR FREISETZUNG VON STICKOXID AUS EINEM POLYMER
APPAREIL, SYSTEME, ET METHODE COMPRENANT UN DONNEUR DE PROTON MICRO-ENCAPSULE POUR LIBERER DE L'OXYDE NITRIQUE D'UN POLYMERE

(30) Priority: 23.08.2005 EP 05018269; 24.08.2005 US 711006 P
(43) Date of publication of application: 07.05.2008
(73) Proprietor: NOLabs AB, 252 25 Helsingborg (SE)
(72) Inventor: PETERS, Tor, CH-8200 Schaffhausen (CH)
(74) Representative: Krahbichler, Erik
(86) International application number: PCT/EP2006/050902
(87) International publication number: WO 2007/023005

(56) References cited:
- WO-A-01/03645
- US-A- 5 519 020
- US-A- 5 888 528
- US-A- 5 910 316

## Description

### Field of the Invention

This invention pertains in general to the field of devices comprising nitric oxide (NO) eluting polymers, involving the use of bound liquid to facilitate and initiate the elution of NO therefrom. More particularly the invention relates to devices manufactured of said NO eluting polymer with bound liquid, and a process for manufacturing of said device.

### Background of the Invention

Nitric oxide (NO) is a highly reactive molecule that is involved in many cell functions. In fact, nitric oxide plays a crucial role in the immune system and is utilized as an effector molecule by macrophages to protect itself against a number of pathogens, such as fungi, viruses, bacteria etc., and general microbial invasion. This improvement of healing is partly caused by NO inhibiting the activation or aggregation of blood platelets, and also by NO causing a reduction of inflammatory processes at the site of an implant.

NO is also known to have an anti-pathogenic, especially an anti-viral, effect, and furthermore NO has an anti-cancerous effect, as it is cytotoxic and cytostatic in therapeutic concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs.

However, due to the short half-life of NO, it has hitherto been very hard to treat viral, bacteria, virus, fungi or yeast infections with NO. This is because NO is actually toxic in high concentrations and has negative effects when applied in too large amounts to the body.

NO is actually also a vasodilator, and too large amounts of NO introduced into the body will cause a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment so far.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible.

Other example for NO eluting polymers are given in US-5,770,645, wherein polymers derivatized with at least one -NOₓ group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of medical devices to be permanently implanted in the body, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO to the tissues surrounding a medical device while minimizing the alteration of the properties of the device. A nanofiber coating, because of the small size and large surface area per unit mass of the nanofibers, provides a much larger surface area per unit mass while minimizing changes in other properties of the device.

When using NO eluting polymers, according to above, in medical applications, said polymers need the presence of water to initiate and facilitate the elution of NO. The present inventor has earlier shown that one way to obtain water or moisture in said usage is to place a water bag or sponge in the vicinity of said NO eluting polymer. This water bag or sponge is then broken to set the polymer in contact with water. One may also use the sweat secreted from the skin underneath the medical application or apply water on the medical application after that said medical application has been placed on the area to be treated.

However, even though the idea is genius, the use of a water bag or sponge presents some disadvantages. The water bags need to be delicate, to facilitate breakage by the person using the medical device. This delicacy aggravates transportation and logistic of said medical devices in some extent. Also, the water bag or sponge is somewhat bulky, which impair logistic and use effectiveness. The use of the secreted sweat presents the problem that not all people sweat sufficiently to obtain an adequate elution of NO at the area to be treated. It is a well known fact that some people sweat more than others. Wetting through sweat is also not a time effective way to obtain enough water and/or moisture, since enough secretion of sweat may take some time to obtain.

US-A-5 519 020 relates to encapsulated NO releasing polymers comprising a source of proton.

Hence, an improved device, or more advantageous, comprising NO eluting polymer, involving the use of bound liquid, such as water or water containing liquid to facilitate and initiate the elution of NO, is needed in the art. It is desired that said liquid is bound in said device in such manner as to eliminate the problems mentioned above in respect.of the prior art, would be advantageous.

### Summary of the Invention

Accordingly, the present invention preferably seeks to mitigate, alleviate or eliminate one or more of the above-identified deficiencies in the art and disadvantages singly or in any combination and solves among others at least the above mentioned problems by providing a device, and a manufacturing method thereof, according to the appended patent claims.

Surprisingly, the present inventor has discovered that it is possible to combine NO eluting polymer and micro encapsulation of a liquid, such as water or a water containing liquid.

Up to this point no one has developed a device comprising an NO eluting polymer and micro encapsulated water or water containing liquid.

In the area of micro encapsulation of liquid, two methods, urea formaldehyde and gelatine capsules, are widely used, but other techniques are also available, which techniques are well known to the skilled artisan. The micro capsules in this technical field may be as small as approximately 8 micrometers and as large as 2 millimeters. They may hold a liquid content of up to approximately 85 %. In this type of micro capsules, the liquid is released by physically rupturing the shell of the micro capsule by pressure, shear forces, or heat.

According to one aspect of the invention, a device is provided comprising an NO eluting polymer and microencapsulated water or water containing liquid, which device may be configured for use as a medical device.

According to another aspect of the invention, a manufacturing process for such a device is provided, wherein the process comprises selecting a plurality of nitric oxide eluting polymeric particles, such as nano fibres, fibres, nano particles, or microspheres, and deploying said nitric oxide eluting particles, and deploying onto said polymeric particles microencapsulated water or water containing liquid.

The present invention has at least the advantage over the prior art that it provides a device that initiates and facilitates elution of NO in a manner that is more prone to withstand transportation, and logistic, and that is pliable to use, hence not bulky.

### Brief Description of the Drawings

These and other aspects, features and advantages of which the invention is capable of will be apparent and elucidated from the following description of embodiments of the present invention, reference being made to the accompanying drawings, in which
Fig. 1 is an illustration of a micro capsule according to an embodiment of the present invention,
Fig. 2 is an illustration of a micro capsule, that has been covered with an NO eluting polymer, according to an embodiment of the invention,
Fig. 3 is an illustration of a mixture of micro capsules and nano-particles or micro spheres according to an embodiment of the present invention,
Fig. 4 is an illustration of a plurality of micro capsules, in for example a film, according to an embodiment of the present invention,
Fig. 5 is an illustration of NO eluting polymer that has been spun onto micro capsules according to an embodiment of the present invention,
Fig. 6 is a planar view of a film of NO eluting polymer that has been combined with a film of micro capsules,
Fig. 7 is a cross-section of a film of NO eluting polymer that has been combined with a film of micro capsules,
Fig. 8 is an illustration of a combination of films, according to Fig. 6 and 7, that has been applied on a target area, and
Fig. 9 illustrates inhibition zone versus patch area.

### Embodiments of the Invention

The following description focuses on embodiments of the present invention applicable to a device, that for example may be configured for medical applications. However, it will be appreciated that the invention is not limited to this application but may be applied to many other technical fields, wherein elution of NO is sought.

With regard to nitric oxide (nitrogen monoxide, NO), its physiological and pharmacological roles have attracted much attention and thus have been studied. NO is synthesized from arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is 2 to 3 orders higher, and that iNOS produces an extremely large amount of NO.

In the case of the generation of a large amount of NO as in the case of the production by iNOS, it is known that NO reacts with active oxygen to attack exogenous microorganisms and cancer cells, but also to cause inflammation and tissue injury. On the other hand, in the case of the generation of a small amount of NO as in the case of the production by cNOS, it is considered that NO takes charge of various protective actions for a living body through cyclic GMP (cGMP), such as vasodilator action, improvement of the blood circulation, anti-platelet-aggregating action, anti-bacterial action, anti-viral action, anti-inflammatory action, anticancer action, acceleration of the absorption at the digestive tract, renal function regulation, neurotransmitting action, erection (reproduction), learning, appetite, and the like. Heretofore, inhibitors of the enzymatic activity of NOS have been examined for the purpose of preventing inflammation and tissue injury, which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

In recent years research has been directed to polymers with the capability of releasing nitrogen oxide when getting in contact with water. Such polymers are for example polyalkyleneimines, such as L-PEI (Linear PolyEthyleneImine) B-PEI (Branched PolyEthyleneImine), PEI-C (PolyEthyleneImine Cellulose), which polymers have the advantage of being biocompatible. Another advantage is that NO is released without any secondary products that could lead to undesired side effects.

"Regulate or control", according to the present invention is intended to be interpreted as the possibility to vary the elution of nitric oxide to thereby achieve different elution profiles.

A polymer comprising an O-nitrosylated group is also a possible nitric oxide eluting polymer. Thus, in one embodiment of the present invention, the nitric oxide eluting polymer comprises diazeniumdiolate groups, S-nitrosylated and O-nitrosylated groups, or any combinations thereof.

In still another embodiment of the present invention said nitric oxide eluting polymer is a poly(alkyleneimine)diazeniumdiolate, such as L-PEI-NO (linear poly(ethyleneimine)diazeniumdiolate), where said nitric oxide eluting polymer is loaded with nitric oxide through the diazeniumdiolate groups and arranged to release nitric oxide at a treatment site.

Some other examples of a suitable nitric oxide eluting polymer are selected from the group comprising amino cellulose, amino dextrans, chitosan, aminated chitosan, polyethyleneimine, PEI-cellulose, polypropyleneimine, polybutyleneimine, polyurethane, poly(buthanediol spermate), poly(iminocarbonate), polypeptide, Carboxy Methyl Cellulose (CMC), polystyrene, poly(vinyl chloride), and polydimethylsiloxane, or any combinations of these, and these mentioned polymers grafted to an inert backbone, such as a polysaccharide backbone or cellulosic backbone.

In still another embodiment of the present invention the nitric oxide eluting polymer may be a O-derivatized NONOate. This kind of polymer often needs an enzymatic reaction to release nitric oxide.

Other ways of describing polymers, which may be suitable as nitric oxide eluting polymer, is polymers comprising secondary amine groups (=N-H), such as L-PEI, or have a secondary amine (=N-H) as a pendant, such as aminocellulose.

The nitric oxide eluting polymer may comprise a secondary amine, either in the backbone or as a pendant, as described previously. This will make a good nitric oxide eluting polymer. The secondary amine should have a strong negative charge to be easy to load with nitric oxide. If there is a ligand close to the secondary amine, such as on a neighbour atom, such as a carbon atom, to the nitrogen atom, with higher electronegativity than nitrogen (N), it is very difficult to load the polymer with nitric oxide. On the other hand, if there is a electropositive ligand close to the secondary amine, such as on a neighbour atom, such as a carbon atom, to the nitrogen atom, the electronegativity of the amine will increase and thereby increase the possibility to load the nitric oxide elution polymer with nitric oxide.

In an embodiment of the present invention the nitric oxide polymer may be stabilized with a salt. Since the nitric oxide eluting group, such as a diazeniumdiolate group, usually is negative, a positive counter ion, such as a cation, may be used to stabilize the nitric oxide eluting group. This cation may for example be selected from the group comprising any cation from group 1 or group 2 in the periodic table, such as Na⁺, K⁺, Li⁺, Be²⁺, Ca²⁺, Mg²⁺, Ba²⁺, and/or Sr²⁺. Different salts of the same nitric oxide eluting polymer have different properties. In this way a suitable salt (or cation) may be selected for different purposes. Examples of cationic stabilized polymers are L-PEI-NO-Na, i.e. L-PEI diazeniumdiolate stabilized with sodium, and L-PEI-NO-Ca, i.e. L-PEI diazeniumdiolate stabilized with calcium.

Three important factors in controlling and regulating the elution of nitric oxide from a nitric oxide eluting polymer are how quickly a proton donor comes in contact with the nitric oxide releasing polymer, such as a diazoliumdiolate group, the acidity of the environment surrounding the nitric oxide eluting polymer, and the temperature of the environment surrounding the nitric oxide releasing polymer (higher temperature promotes elution of nitric oxide).

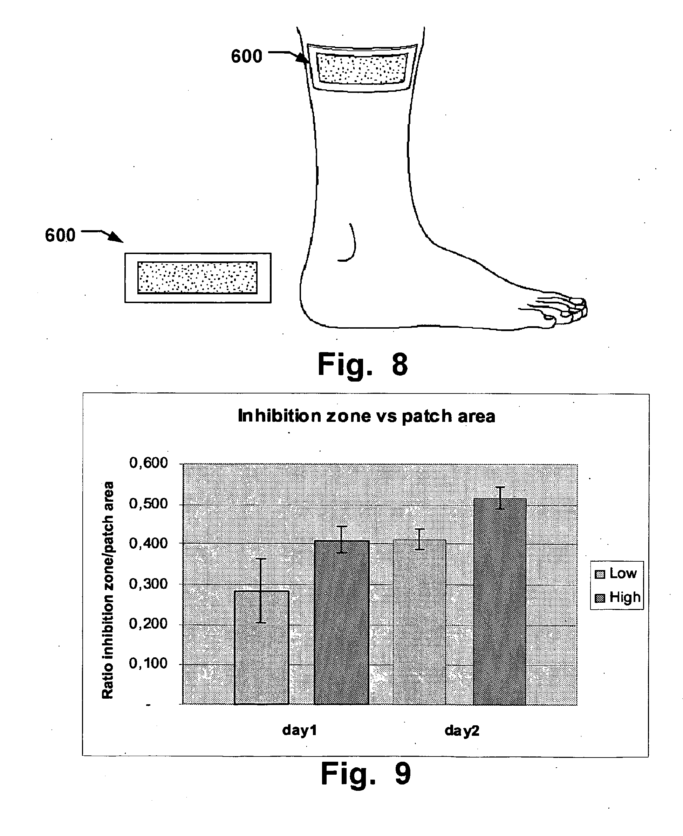

In one embodiment of the present invention a nitric oxide eluting polymer, such as L-PEI-NO, is mixed with a carrier polymer to slow down or prolong the elution of nitric oxide. Also, in another embodiment, the nitric oxide eluting polymer may be mixed with more than one carrier polymer, whereby be elution or release may be tailor made to fit specific needs. Such a need may for example be a low elution during a first period of time, when the environment of the nitric oxide eluting polymer is hydrophobic, and a faster elution during a second period of time, when the environment of the nitric oxide eluting polymer has been altered to be more hydrophilic. This may for example be accomplished by using biodegradable polymers, whereby a low elution during a first period of time is obtained, after which, when the hydrophobic polymer has been dissolved, the hydrophilic polymer provides a higher elution of nitric oxide. Thus, a more hydrophobic carrier polymer will give a slower elution of nitric oxide, since the activating proton donor, such as water or body fluid, will penetrate the carrier polymer slower. On the other hand, a hydrophilic polymer acts the opposite way. One example of an hydrophilic polymer is polyethylene oxide, and one example of an hydrophobic polymer is polystyrene. These carrier polymers may be mixed with the nitric oxide eluting polymer and then electrospun to suitable fibers. The skilled person in the art knows which other polymers may be used for similar purposes. Fig. 9 illustrates two elution profiles (NO concentration vs. time) for two different polymer - mixtures; a nitric oxide eluting polymer mixed with a hydrophilic carrier polymer in an acidic environment (A), and a nitric oxide eluting polymer mixed with a hydrophobic carrier polymer in a neutral environment (B).

In one embodiment this carrier polymer is substituted by another material with hydrophobic or hydrophilic properties. Therefore, the term "carrier material" in the present context should be interpreted to include carrier polymers and other materials with hydrophilic or hydrophobic properties.

In another embodiment of the present invention the elution of nitric oxide from a nitric oxide eluting polymer, such as L-PEI-NO, is influenced by the presence of protons. This means that a more acidic environment provides a quicker elution of nitric oxide. By activating the nitric oxide eluting polymer, or mixture of nitric oxide eluting polymer and carrier material, with an acidic fluid, such as an ascorbic acid solution, the elution of nitric oxide may be accelerated.

The carrier polymers and carrier materials mentioned above may affect other characteristics than the regulation of nitric oxide elution. An examples of such characteristic is mechanical strength.

In respect of the carrier polymers or carrier materials, the NO-eluting polymer may be integrated in, spun together with, or spun on top of, any of these materials in all of the embodiments of the present invention. This spinning includes electro spinning, air spinning, dry spinning, wet spinning, melt spinning, and gel spinning. In this way, one may manufacture fibers of a polymer mixture, comprising a nitric oxide eluting polymer and a carrier polymer, or a carrier material, with predefined nitric oxide eluting characteristics. These characteristics may be tailor made for different elution profiles in different applications.

The polymers may be manufactured by electro spinning, gas spinning, air spinning, wet spinning, dry spinning, melt spinning, and gel spinning. Electro spinning is a process by which a suspended polymer is charged. At a characteristic voltage a fine jet of polymer releases from the surface in response to the tensile forces generated by interaction by an applied electric field with the electrical charge carried by the jet. This process produces a bundle of polymer fibres, such as nano-fibres. This jet of polymer fibres may be directed to a surface to be treated.

Furthermore, US 6,382,526, US 6,520,425, and US 6,695,992 disclose processes and apparatuses for the production of such polymeric fibres. These techniques are generally based on gas stream spinning, also known within the fiber forming industry as air spinning, of liquids and/or solutions capable of forming fibers.

Other example for NO eluting polymers are given in US-5,770,645, wherein polymers derivatized with at least one -NOX group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of permanently implanted medical devices, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner.

However, the meaning of "controlled" in the context of US 6,737,447 is only directed to the fact that nitric oxide is eluted from the coating during a period of time, i.e that the nitric oxide not is eluted all in once. Therefore, the interpretation of "controlled" in respect of US 6,737,447 is different from the meaning of "regulating" in the present invention.

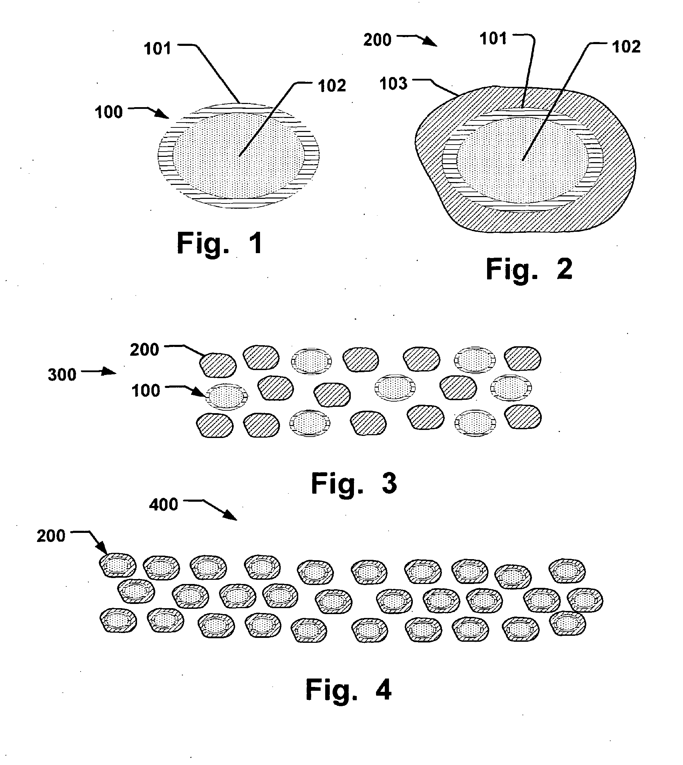

In one embodiment of the present invention an NO eluting polymer, such as polyalkyleneimines, such as L-PEI (Linear PolyEthyleneImine) B-PEI (Branched PolyEthyleneImine), PEI-C (PolyEthyleneImine Cellulose), is provided, and/or combined, with microencapsulated liquid, such as water or water containing liquid, according to Fig. 1. Fig. 1 shows a microcapsule 100, comprising a shell 101 and a microencapsulated liquid 102, such as water or water containing liquid.

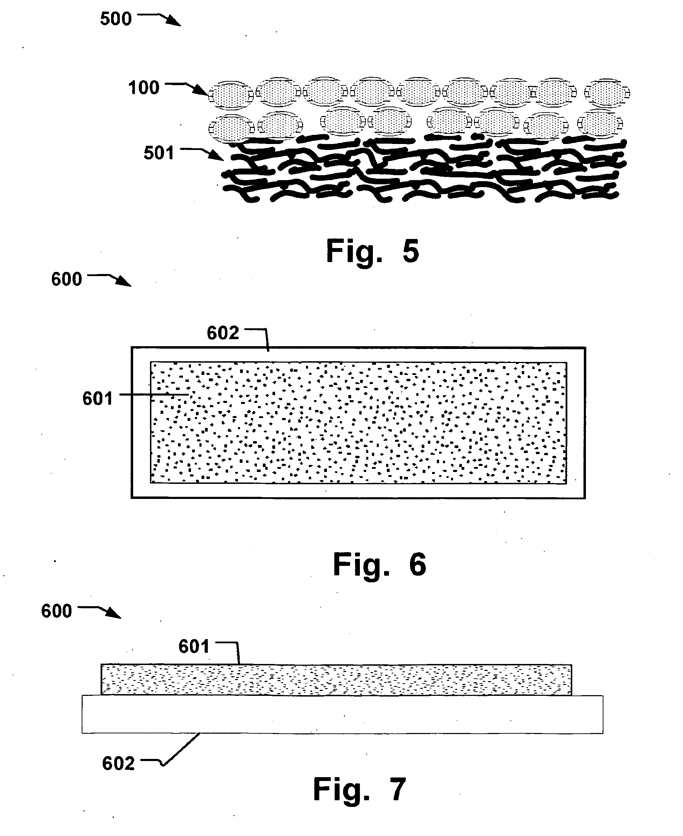

This may for example be done by first manufacture micro capsules, containing water or water containing liquid, in a state of the art manner. These micro capsules may then be formed into a film, tape, sheath, etc. These micro capsules, in form of a film, tape, sheath, etc., are then applied on the NO eluting polymer, according to Fig. 6, which is a planar view of an NO eluting polymer any a film, etc., of micro capsules, and Fig. 7, which is a cross section of the configuration in Fig. 6. The application of the micro capsules on the NO eluting polymer may for example be done by gluing, such as pattern gluing, or instead spinning the NO eluting polymer onto said micro capsules. In this way a device comprising NO eluting polymer and micro encapsulated water or water containing liquid is manufactured. Said device may for example be any device selected from the group; patches, ointments, tapes for cosmetic treatment; tapes, condoms, patches, sheets for treatment of wounds or infections in the oral cavity; patches, socks, condoms for treatment of onychomycosis; patches, socks, tapes, sheets for treatment and/or prevention of neuropathy, such as diabetic neuropathy, diabetic ulcers, vaso-constrictive disorders and macro-angiopathy; condoms, sheets, patches for treatment of rectal disorders, such as fissures, ulcers, haemorrhoids, and levator spasm; devices for target treatment of gastric and gastrointestinal complications, such as gastric ulcer; condom/sheath, tape/coating, fibres, nano-particles, or micro-spheres for wound care; devices for prevention of infection and obtainment of antithrombotic effect; feedstuff or food; and patches etc., for pre-treatment of an area before insertion of a catheter, venflone etc. Said device may for example be any of which are mentioned in the pending European patent applications; 04029796.2, 05006474.0, 05002937.0, 05002935.4, 05002934.7, 05002933.9, 05006495.5, 05006489.8, 05011785.2, and 05011786.0, which pending European patent applications are based on inventions made of the present inventor, and which pending applications hereby are integrated as references in the present application.

Of course, in other embodiments of the present invention, the liquid contained in the micro capsules may be any other proton donor, such as,water, body fluids. (blood, lymph, bile, etc.), alcohols (methanol, ethanol, propanols, buthanols, pentanols, hexanols, phenols, naphtols, polyols, etc.), aqueous acidic buffers (phosphates, succinates, carbonates, acetates, formats, propionates, butyrates, fatty acids, amino acids, etc.), or any combinations of these, or any other polar solvent, with the ability to initiate and produce elution of NO from said NO eluting polymers.

In another embodiment a substance that changes color when it comes in contact with water can be incorporated in the device. Thus when the water capsules or water bag breaks the material changes color, thereby indicating that the material is activated.

In another embodiment of the present invention the device only allows NO-elution in one direction. In this kind of embodiment one side of the device has low permeability, or substantially no permeability, to nitric oxide. This may also be accomplished by applying a material on one side of the device according to the invention that is not permeable to NO. Such materials may be chosen from the group comprising common plastics, such as fluoropolymers, polyethylene, polypropylene, polyacrylonitrile, polyurethane, polyvinylacetates, polylacticacids, starch, cellulose, polyhydroxyalkanoates, polyesters, polycaprolactone, polyvinylalcohol, polystyrene, polyethers, polycarbonates, polyamides, poly(acrylic acid), Carboxy Methyl Cellulose (CMC), protein based polymers, gelatine, biodegradable polymers, cotton, and latex, or any combinations of these. This embodiment is also easy to manufacture as the NO eluting polymer, e.g. L-PEI (or nitric oxide eluting polymer and carrier material, which will be explained in more detail below) may be electro or gas-jet spun onto the surface of the device according to the invention of e.g. the mentioned plastics, latex, or cotton.

In still another embodiment the device is provided with one membrane, which is permeable to nitric oxide, on a first side of the device, and another membrane, which has low permeability or substantially no permeability to nitric oxide, on a second side of said device. This embodiment provides the possibility to direct the elution to said first side of the device, while the elution of nitric oxide is substantially prevented from said second side. Thereby, a greater amount of nitric oxide will reach the intended area to be treated.

The activation of the nitric oxide eluting polymer may be accomplished by contacting said polymer with a suitable proton donor. In one embodiment the proton donor may be selected from the group comprising water, body fluids (blood, lymph, bile, etc.), alcohols (methanol, ethanol, propanols, buthanols, pentanols, hexanols, phenols, naphtols, polyols, etc.), aqueous acidic buffers (phosphates, succinates, carbonates, acetates, formats, propionates, butyrates, fatty acids, amino acids, etc.), or any combinations of these.

By adding a surfactant in the proton donor one can facilitate the wettening of the device. The surfactant lowers the surface tension and the activating fluid is easily transported throughout the device.

Another embodiment of the present invention comprises mixing the nitric oxide eluting polymer, or a mixture of the nitric oxide eluting polymer and a carrier material, with an absorbent agent. This embodiment provides the advantage of an accelerated elution of nitric oxide since the polymer, or polymer mixture, via the absorbent agent, may take up the activating fluid, such as water or body fluid, much faster. In one example 80 % (w/w) absorbent agent is mixed with the nitric oxide eluting polymer, or mixture of nitric oxide eluting polymer and carrier material, and in another embodiment 10 to 50 % (w/w) absorbent agent is mixed with the nitric oxide eluting polymer, or mixture of nitric oxide eluting polymer and carrier material.

Since the elution of nitric oxide is activated by a proton donor, such as water, it may be an advantage to keep the nitric oxide eluting polymer, or mixture of nitric oxide eluting polymer and carrier material, in contact with said proton donor. If an indication requires an elution of nitric oxide during a prolonged period of time, a system is advantageous, which presents the possibility to keep the proton donor in contact with the nitric oxide eluting polymer, or mixture of nitric oxide eluting polymer and carrier material. Therefore, in still another embodiment of the present invention, the elution of nitric oxide may be regulated by adding an absorbent agent. The absorbent agent absorbs the proton donor, such as water, and keeps the proton donor in close contact with the nitric oxide eluting polymer during prolonged periods of time. Said absorbent agent may be selected from the group comprising polyacrylates, polyethylene oxide, carboxymethylcellulose, and microcrystalline cellulose, cotton, and starch. This absorbent agent may also be used as a filling agent. In this case said filling agent may give the nitric oxide eluting polymer, or mixture of said nitric oxide eluting polymer and a carrier material, a desired texture.

The device may then be applied on a target area on which exposure of NO is desired. Such a target area may for example be located on an animal organ, such as the skin, mucous membrane etc, or any other area mentioned and/or described in the pending European patent applications mentioned above.

When the device is applied on the target area the device or system is compressed or squeezed. Said compression or squeezing results in breakage of the micro capsules. The NO eluting polymer is thus exposed to said water or water containing liquid, and the elution of NO from the NO eluting polymer is initiated on the target area.

In other embodiments of the present invention the liquid inside the micro capsules is released by heating or shearing the micro capsules until the micro capsules are ruptured.

The elution of NO from said polymer may be used for any conceivable purpose, such as to obtain anti microbial and/or viral effect, vasodilating effect, anti fungal effect, etc.

In another embodiment of the present invention microcapsules, containing water or water containing liquid, are manufactured in a manner according the state of the art. These micro capsules are then covered with an NO eluting polymer, according to above. The covering of the micro capsules is for instance done by spinning the NO eluting polymer onto the micro capsules, containing water or water containing liquid, according to Fig. 2, in which an NO eluting polymer 103 encloses a microcapsule 101. When the combined particle 200 is compressed, or in any other way ruptured, the liquid, such as water or water containing liquid, will get in contact with the NO eluting polymer 103, and thus the elution of NO is initiated. The particles 200 may for example constitute a film, sheath, tape, etc., such as illustrated in Fig. 4.

The spinning may for example be done by air spinning, electro spinning, gas spinning, wet spinning, dry spinning, melt spinning, or gel spinning. In this way microcapsules covered with NO eluting polymer may be manufactured.

In other embodiments of the invention, the NO eluting polymer may be mixed and manufactured together with other suitable materials, such as polyethylene, polypropylene, polyacrylonitrile, polyurethane, polyvinylacetates, polylacticacids, starch, cellulose, polyhydroxyalkanoates, polyesters, polycaprolactone, polyvinylalcohol, polystyrene, polyethers, polycarbonates, polyamides, polyolefins, poly(acrylic acid), Carboxy Methyl Cellulose (CMC), protein based polymers, gelatine, biodegradable polymers, cotton, and latex, or any combinations of these. The NO-eluting polymer may be integrated in, spun together with, or spun on top of, any of these materials in all of the embodiments of the present invention.. In these embodiments the elution of NO is regulated, such as by decreasing the elution rate, by the admixed materials.

In an embodiment of the invention the NO eluting polymer is in form of nano-particles, or micro spheres. These nano-particles, or micro-spheres, may be formed from the NO-eluting polymers by grinding or in any other way divide the spun polymeric fibres into small parts.

In another embodiment of the device said device may be manufactured in the form of a polyurethane, or polyethylene, tape or coating. This polyurethane tape or coating may easily be wrapped around, or applied on, the target area to be treated. At least the side facing the body may be covered with NO-eluting nano-particles, or micro-spheres, or nano-filament of NO-eluting polymer. The covering of NO-eluting nano-particles, or micro-spheres, or nano-filament of NO-eluting polymer is in turn covered with the micro capsules, containing water or water containing liquid. When these particles or filaments get in contact with the water, moisture or water containing liquid inside the micro capsules, after the micro capsules have been compressed or squeezed until the micro capsules break and the water or water containing liquid inside the micro capsules is let out, the NO eluting polymer starts to elute NO.

The increased blood perfusion and vasodilatation, that may obtained from the device may in another embodiment of the present invention, result in an improved effect when combined with other products, comprising active components. Thus, the synergistic effect from NO and other wound healing, or anti-microbial, anti-inflammatory, or anti-viral, components is within the scope of the present invention.

These fibres, nano-particles, or micro-spheres, may in one embodiment be formed from the NO-eluting polymers comprised in the present invention, for example polyalkyleneimines, such as L-PEI (Linear PolyEthyleneImine), B-PEI (Branched PolyEthyleneImine), and PEI-C (PolyEthyleneImine Cellulose), which polymers have the advantage of being biocompatible. They may also be encapsulated in any suitable material, such as polyethylene, polypropylene, polyacrylonitrile, polyurethane, polyvinylacetates, polylacticacids, starch, cellulose, polyhydroxyalkanoates, polyesters, polycaprolactone, polyvinylalcohol, polystyrene, polyethers, polycarbonates, polyamides, polyolefins, poly(acrylic acid), Carboxy Methyl Cellulose (CMC), protein based polymers, gelatine, biodegradable polymers, cotton, and latex, or any combinations of these. In the context of this embodiment the term "encapsulate" is intended to be interpreted as fixating the nitric oxide eluting polymer in a three dimensional matrix such as a foam, a film, a nonwoven mat of nano-fibers, fibers, or other materials with the capability to fixate the NO eluting polymer, or enclosing the nitric oxide eluting polymer in any suitable material. Thus, the term "encapsulate" in this embodiment should not be confused with the terms "micro encapsulate" or "micro encapsulation" used in the description of the present invention.

In a further embodiment fibres, nano-particles, or micro-spheres of an NO eluting polymer are mixed with micro capsules, containing water or water containing liquid, according to Fig. 3, wherein fibres, nano-particles, or micro-spheres 200 of an NO eluting polymer are mixed with micro capsules 100. The mixture 300 is then for example applied on a carrier material, such as a tape of polyethylene or any other suitable carrier material. From this tape patches, sheets, or the like, are constructed, which patches, sheets, or the like then are applied on the target area to which elution of NO is desired. It is also possible to produce a film, tape, etc., directly from a mixture of fibres, nano-particles, or micro-spheres 200 and the micro capsules 100.

In still another embodiment, according to Fig. 6 and 7, the micro capsules, containing water or water containing liquid, are formed into a film, tape, or sheath 602. Thereafter, a film, tape, or sheath of an NO eluting polymer 601 is glued onto the film, tape, or sheath of micro capsules 602, containing water or water containing liquid. Preferably the film, tape, or sheath of the NO eluting polymer 601 is glued onto the film, tape, or sheath of the micro capsules, containing water or water containing liquid, in patterned way. The obtained pattern includes spaces where there is no glue, in which spaces the water or water containing liquid will be transported to the NO eluting polymer once the micro capsules are broken from compression or squeezing. When the water or water containing liquid gets in contact with the NO eluting polymer the elution of NO starts. Thus, the combination of film, tape, or sheath of micro capsules, containing water or water containing liquid, and NO eluting polymer may be applied on a target area, such as in Fig. 8. Thereafter the combination is compressed or squeezed, which results in that the target area is exposed to NO.

In yet another embodiment the NO eluting polymer is spun directly onto the film, tape, or sheath of micro capsules, containing water or water containing liquid, according to Fig. 5, in which fibres 501 of an NO eluting polymer are spun onto the micro capsules 100. The combination of film, tape, or sheath of micro capsules, containing water or water containing liquid, and spun NO eluting polymer may be applied on a target area. Thereafter the combination is compressed or squeezed, which results in that the target area is exposed to NO.

In still another embodiment of the present invention the device is provided with an activation indicator. This activation indicator indicates when the micro capsules are satisfyingly broken, hence when the NO eluting polymer is subjected to enough water or water containing liquid to elute an efficient amount of NO. This activation indicator may for example be obtained by coloring the water or water containing liquid that is trapped inside the micro capsules. When the micro capsules are broken the colored water or water containing liquid escapes the microcapsules and the color gets visualized while efficiently wetting the NO eluting polymer. Another way of obtaining an activation indicator is to choose a manufacture the micro capsules in a material, or choose a wall thickness of said micro particles, that creates a sound when the micro capsules break. It is also possible to admix a scent in the water or water containing liquid, contained in the micro capsules. This results in that the user of the device may smell the scent when the water or water containing liquid escapes from the micro capsules after breakage thereof. The released NO may even synergetically augment this scent impression, by itself or by influencing the smell sensing organs, e.g. by vasodilation thereof.

In another embodiment of the present invention the device only allows NO-elution in one direction. In this kind of embodiment one side of the device according to the invention is non-permeable to NO. This may be accomplished by applying a material on one side of the device according to the invention that is not permeable to NO. Such materials may be chosen from the group comprising common plastics, such as polyethylene, polyurethane etc. This embodiment is also easy to manufacture as the NO eluting polymer, e.g. L-PEI nano fibres may be electro or gas-jet spun onto the surface of the device according to the invention of e.g. the mentioned plastics, latex, or cotton.

In yet another embodiment of the present invention the NO-eluting device is acting as a booster for drug eluting patches, e.g. pharmaceuticals, vitamins, nicotin, nitroglycerin, Non-Steroidal Anti-Inflammatory Drugs (NSAID), such as diclofenac, ibuprofen, aspirin, naproxen, COX-2 inhibitors, choline magnesium trisalicylate, diflunisal, salsalate, fenoprofen, flurbiprofen, ketoprofen, oxaprozin, indomethacin, sulindac, tolmetin, meloxicam, piroxicam, meclofenamate, mefenamic acid, nabumetone, etodalac, ketorolac, celecoxib, valdecoxib, and rofecoxib; steroids, such as cortisone, prednisone, methylprednisolone, prednisolone, vitamin D, estrogen, cholestrol, beclomethasone, flunisolide, fluticasone, triamcinolone, desonide, clobetasol, alclometasole, desoximetasone, betamethasone, halcinonide and dexamethasone; pain reliefs, such as motrin, feldene, naprosyn, lidocaine, and prilocaine; and other substances, such as indinavirsulfate, finasteride, aprepitant, montelukast sodium, alendronate sodium, rofecoxib, rizatriptan benzoate, simvastatin, finasteride, ezetimibe, caspofungin acetate, ertapenem sodium, dorzolamide hydrochloride, timolol maleate, losartan potassium, and hydrochlorotiazide; etc. This embodiment presents a device with the advantage of combining two treatments, of significant value, in one treatment.

Hence, when the device is used as a medical application, such device may achieve a synergetic effect, when NO is eluted from said device. NO has a vasodilatory effect on the region where the device having the combination compound actuates. Vasodilated tissue is more susceptible to certain medications and thus more easily treated by the medical preparations and still NO has in addition to that the anti-inflammatory, anti-bacterial etc. effect. Hence, an unexpected surprisingly effective treatment is provided.

The device elutes nitric oxide (NO) from said eluting polymer in a therapeutic dose, such as between The device elutes nitric oxide (NO) from said eluting polymer in a therapeutic dose, such as between 0.001 to 5000 ppm, such as 0.01 to 3000 ppm, such as 0.1 to 1000 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm. The concentration may vary widely depending on where the concentration is measured. If the concentration is measured close to the actual NO eluting polymer the concentration may be as high as thousands of ppm, while the concentration inside the tissue in this case often is considerably lower, such as between 1 to 1000 ppm.

In the embodiments of the present invention it may be suitable to control or regulate the time span of NO release from the device according to the invention. This may be accomplished by integrating other polymers or materials in said device. These polymers or materials may be chosen from any suitable material or polymer, such as polyethylene, polypropylene, polyacrylonitrile, polyurethane, polyvinylacetates, polylacticacids, starch, cellulose, polyhydroxyalkanoates, polyesters, polycaprolactone, polyvinylalcohol, polystyrene, polyethers, polycarbonates, polyamides, polyolefins, poly(acrylic acid), Carboxy Methyl Cellulose (CMC), protein based polymers, gelatine, biodegradable polymers, cotton, and latex, or any combinations of these.

The NO-eluting polymers in the device may be combined with silver, such as hydroactivated silver. The integration of silver in the devices gives the healing process an extra boost. Preferably the silver is releasable from the devices in the form of silver ions. The integration of silver in the device may present several advantages. One example of such an advantage is that the silver may keep the device in itself free from bacteria or viruses, while the nitric oxide eluting polymer elutes the therapeutic dosage of nitric oxide to the target site.

The device may be manufactured by, for example electro spinning of L-PEI or other polymers comprising L-PEI or being arranged in combination with L-PEI. L-PEI is the charged at a characteristic voltage, and a fine jet of L-PEI releases as a bundle of L-PEI polymer fibres. This jet of polymer fibres may be directed to a surface to be treated. The surface to be treated may for example be any suitable material. The electro spun fibres of L-PEI then attach on said material and form a coating/layer of L-PEI on the device according to the invention.

It is of course possible to electro spin the other NO-eluting polymers, according to above, on the device according to the invention while still being inside the scope of the present invention.

In one embodiment the NO-eluting polymers are electro spun in such way that pure NO-eluting polymer fibres may be obtained.

It is also within the scope of the present invention to electro spin an NO-eluting polymer together with other suitable polymer/polymers.

Gas stream spinning, dry spinning, wet spinning, melt spinning, gel spinning, or air spinning, of said NO-eluting polymers onto a film of microencapsulated water or water containing liquid or a combination of microencapsulated water or water containing liquid and any suitable NO eluting or non NO eluting polymer is also within the scope of the present invention.

The manufacturing process presents the advantages of large contact surface of the NO-eluting polymer fibres with the area to be covered with NO eluting polymer, effective use of NO-eluting polymer, and a cost effective way of producing the device.

Hereinafter, some potential uses of the present invention are described:

A method of treating an animal organ, comprising applying a device, that comprises a nitric oxide (NO) eluting polymer configured for eluting a therapeutic dosage of nitrogen oxide (NO) when used for said treatment and micro capsules, containing water or water containing liquid, rupturing said micro capsules to set said water or water containing liquid in contact with said NO eluting polymer, and thereby exposing said organ to said nitric oxide when said polymer in use elutes nitrogen oxide (NO) by eluting a therapeutic dose of nitric oxide from said nitric oxide eluting polymer to said treatment site.

The method according to above, wherein said site of said at least one wound is a head, face, neck, shoulder, back, arm, hand, stomach, genital, thigh, leg, or foot of an animal, such as a human, of a body, and wherein said method comprises applying a device, according to above, to said head, face, neck, shoulder, back, arm, hand, stomach, genital, thigh, leg, or foot, for said exposure.

Use of nitric oxide (NO) in a therapeutic dose for therapeutically treating and/or preventing at least one part of an organ.

The invention may be implemented in any suitable form. The elements and components of the embodiments according to the invention may be physically, functionally, and logically implemented in any suitable way. Indeed, the functionality may be implemented in a single unit, in a plurality of units, or as part of other functional units.

In the claims, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A medical device, comprising a nitric oxide (NO) eluting polymer configured for elution of nitric oxide (NO) therefrom upon contact between a proton donor and said nitric oxide (NO) eluting polymer,
wherein said device is adapted to be applied on a target area on which exposure of nitric oxide (NO) is desired, and wherein said device is chosen from a patch, ointment, tape, sock, condom, or sheet;
**characterized in that**
said device is provided with a proton donor configured for said contact, and which is microencapsulated in micro capsules, and wherein
said micro capsules, in which said proton donor is contained, are arranged to release at least a part of said proton donor after breakage of said micro capsules, and
said micro capsules are arranged such that said proton donor, when released after said breakage, at least partly contacts said nitric oxide (NO) eluting polymer such that elution of nitric oxide (NO) from said nitric oxide (NO) eluting polymer is initiated, whereby said elution of nitric oxide (NO) from said nitric oxide (NO) eluting polymer in use of said device is provided on said target area.

2. The medical device according to claim 1, wherein said nitric oxide (NO) eluting polymer comprises diazeniumdiolate groups, S-nitrosylated groups, and O-nitrosylated groups, or any combination of these.

3. The medical device according to claim 1 or 2, wherein said nitric oxide (NO) eluting polymer is L-PEI (linear polyethyleneimine), loaded with nitric oxide (NO) through said diazeniumdiolate groups, S-nitrosylated groups, or O-nitrosylated groups, or any combination of these.

4. The medical device according to claim 1, 2, or 3, wherein said nitric oxide eluting polymer is selected from the group comprising amino cellulose, amino dextrans, chitosan, aminated chitosan, polyethyleneimine, PEI-cellulose, polypropyleneimine, polybutyleneimine, polyurethane, poly(buthanediol spermate), poly(iminocarbonate), polypeptide, Carboxy Methyl Cellulose (CMC), polystyrene, poly(vinyl chloride), and polydimethylsiloxane, or any combinations of these, and these mentioned polymers grafted to an inert backbone, such as a polysaccharide backbone or cellulosic backbone.

5. The medical device according to claim 1, 2, 3, or 4, wherein said proton donor is selected from the group comprising water, blood, lymph, bile, methanol, ethanol, propanols, buthanols, pentanols, hexanols, phenols, naphtols, polyols, phosphates, succinates, carbonates, acetates, formats, propionates, butyrates, fatty acids, and amino acids, or any combinations of these.

6. The medical device according to claim 1, wherein said microencapsulated proton donor is microencapsulated in formaldehyde and gelatine microcapsules.

7. The medical device according to claim 1, wherein said device comprises a film comprising said proton donor microencapsulated in said microcapsules, wherein said NO eluting polymer is spun onto said film.

8. The medical device according to claim 1, wherein said device comprises said NO eluting polymer mixed with said microcapsules containing said proton donor.

9. The medical device according to claim 8, wherein said NO eluting polymer, configured to elute NO, is provided in form of fibres, nano-particles and/or micro-spheres.

10. The medical device according to claim 1, wherein said NO eluting polymer is provided in form of a film, sheath or tape, which is attached onto a film, sheath, or tape comprising said proton donor, microencapsulated in said microcapsules.

11. The medical device according to claim 1, wherein said NO eluting polymer comprises a secondary amine in a backbone or a secondary amine as a pendant.

12. The medical device according to claim 11, wherein a positive ligand is located on a neighbor carbon atom to the secondary amine.

13. The medical device according to claim 1, comprising an absorbent agent.

14. The medical device according to claim 13, wherein said absorbent agent is selected from the group comprising polyacrylate, polyethylene oxide, Carboxy Methyl Cellulose (CMC), microcrystalline cellulose, cotton, or starch, or any combinations thereof.

15. The medical device according to claim 1 or 13, comprising a cation for stabilizing the nitric oxide eluting polymer.

16. The medical device according to claim 15, wherein said cation is selected from the group comprising Na⁺, K⁺, Li⁺, Be²⁺, Ca²⁺, Mg²⁺, Ba²⁺, and/or Sr²⁺, or any combinations thereof.

17. The medical device according to claim 10, wherein said attachment is performed with glue.

18. The medical device according to claim 17, wherein said glue is applied in a pattern allowing for the proton donor inside said micro capsules to get in contact with said NO eluting polymer after breakage of said micro capsules.

19. The medical device according to claim 1, wherein said device is supplied with an activation indicator.

20. The medical device according to claim 19, wherein said activation indicator is in form of a color, scent, and/or sound indicator.

21. The medical device according to claim 1, wherein one side of the device has low permeability, or substantially no permeability, to nitric oxide.

22. The medical device according to claim 21, wherein the device is provided with one membrane, which is permeable to nitric oxide, on a first side of the device, and another membrane, which has low permeability or substantially no permeability to nitric oxide, on a second side of said device.

23. A manufacturing process for a medical device according to claim 1, comprising:
selecting a plurality of nitric oxide (NO) eluting polymeric particles, including nano fibres, nano particles or micro spheres,
microencapsulating a proton donor to form micro capsules containing said proton donor,
applying said micro capsules on said nitric oxide (NO) eluting polymer, to form said device.

24. The manufacturing process according to claim 23, further comprising
selecting said nitric oxide (NO) eluting polymer such that it is configured to elute a therapeutic dosage of nitric oxide (NO),
selecting a carrier material, which carrier material is configured to regulate and control the elution of said therapeutic dosage of nitric oxide (NO),
incorporating the NO-eluting polymer with said carrier material into an nitric oxide (NO) eluting material, such that said carrier material, in use of said device, regulates and controls the elution of said therapeutic dosage of nitric oxide (NO), and
deploying said nitric oxide eluting material into a suitable form, or as a coating onto a carrier, to form at least a part of said device, such that said device is configured to expose a therapeutic target site to said nitric oxide when said NO-eluting polymer in use elutes nitric oxide (NO).

25. The manufacturing process according to claim 24, wherein said selecting said nitric oxide (NO) eluting polymer comprises selecting a plurality of nitric oxide (NO) eluting polymeric particles, preferably nano fibres, nano particles or micro spheres.

26. The manufacturing process according to claim 24 or 23, wherein said incorporating said NO-eluting polymer with said carrier material comprises integrating said NO-eluting polymer in said carrier material, spinning said NO-eluting polymer together with said carrier material, or spinning said NO-eluting polymer on top of said carrier material, in order to predefine nitric oxide eluting characteristics of said device.

27. The manufacturing process according to claim 23, further comprising microencapsulating said proton donor in said micro capsules, prior to deploying said nitric oxide (NO) eluting polymer.

28. The manufacturing process according to claim 23, wherein said applying comprises pattern gluing, or spinning the NO eluting polymer onto said micro capsules.

29. The manufacturing process according to claim 23, comprising forming the micro capsules into a first film, tape, or sheath,
forming a second film, tape, or sheath comprising said NO eluting polymer, and
gluing the first film, tape, or sheath of micro capsules to said second film, tape, or sheath comprising said NO eluting polymer.

30. The manufacturing process according to claim 29, wherein said gluing comprises patterned gluing, such that a pattern is obtained including glue free spaces.

31. The manufacturing process according to claim 23, comprising forming the micro capsules into a first film, tape, or sheath, and directly spinning a material comprising the NO eluting polymer onto the film, tape, or sheath of micro capsules, containing a proton donor.

32. The manufacturing process according to claim 23, comprising providing an activation indicator configured to indicate when the micro capsules are broken such that the NO eluting polymer is subjected to said proton donor to elute NO.

33. The manufacturing process according to claim 32, wherein said providing an activation indicator comprises providing a coloring agent inside the micro capsules.

34. The manufacturing process according to claim 32, wherein said providing an activation indicator comprises selecting a material for the micro capsules, or choosing a wall thickness of said micro capsules, that creates a sound when the micro capsules break.

35. The manufacturing process according to claim 32, wherein said providing an activation indicator comprises admixing a scent material into the micro capsules.

36. The manufacturing process according to claim 32, wherein said providing an activation indicator comprises providing a substance that changes color when it comes in contact with the proton donor.

37. A method of activating nitric oxide (NO) elution from a medical device according to claim 1, said device comprising a NO eluting polymer configured to elute nitric oxide (NO) therefrom upon contact with a proton donor, comprising
arranging said NO eluting polymer in the vicinity of micro capsules containing said proton donor, and
releasing said proton donor by rupturing said micro capsules for contacting said NO eluting polymer with said proton donor.

38. The method according to claim 37, wherein said rupturing is performed with pressure, shear, or heat.

39. A nitric oxide (NO) eluting polymer for use in a medical device or system for treating an organ of a body of an animal, such as a human,
wherein the medical device comprises the nitric oxide (NO) eluting polymer which is configured for eluting a therapeutic dosage of nitric oxide (NO) when used for said treatment and micro capsules, containing a proton donor containing liquid, including water or water containing liquid, arranged to rupture to set said proton donor containing liquid in contact with said NO eluting polymer, and arranged to expose said organ to said nitric oxide when said polymer in use elutes nitric oxide (NO) by eluting said therapeutic dose of nitric oxide from said nitric oxide eluting polymer to said organ.

## Patentansprüche

1. Medizinische Vorrichtung, umfassend ein Stickoxid (NO) eluierendes Polymer, das ausgelegt ist, um bei Kontakt zwischen einem Protonenspender und dem Stickoxid (NO) eluierenden Polymer Stickoxid (NO) daraus zu eluieren,
wobei die Vorrichtung angepasst ist, um auf eine Zielfläche angewendet zu werden, auf der die Freisetzung von Stickoxid (NO) gewünscht ist, und wobei die Vorrichtung ausgewählt wird aus einem Pflaster, einer Salbe, einem Klebeband, einem Strumpf, einem Kondom oder einem Tupfer;
**dadurch gekennzeichnet, dass**
die Vorrichtung mit einem Protonenspender versehen ist, der für diesen Kontakt ausgelegt ist und in Mikrokapseln mikroverkapselt ist, und wobei
die Mikrokapseln, in denen der Protonenspender enthalten ist, angeordnet sind, um zumindest einen Teil des Protonenspenders nach dem Zerbrechen der Mikrokapseln freizusetzen, und
die Mikrokapseln so angeordnet sind, dass der Protonenspender, wenn er nach dem Zerbrechen freigesetzt ist, zumindest teilweise mit dem Stickoxid (NO) eluierenden Polymer in Kontakt kommt, so dass die Elution von Stickoxid (NO) aus dem Stickoxid (NO) eluierenden Polymer initiiert wird, wodurch bei Verwendung der Vorrichtung auf der Zielfläche die Elution des Stickoxids (NO) aus dem Stickoxid (NO) eluierenden Polymer erfolgt.

2. Medizinische Vorrichtung nach Anspruch 1, wobei das Stickoxid (NO) eluierende Polymer Diazeniumdiolatgruppen, S-nitrosylierte Gruppen und O-nitrosylierte Gruppen oder beliebige Kombinationen davon umfasst.

3. Medizinische Vorrichtung nach Anspruch 1 oder 2, wobei das Stickoxid (NO) eluierende Polymer L-PEI (lineares Polyethylenimin) ist, das durch die Diazeniumdiolatgruppen, S-nitrosylierten Gruppen oder O-nitrosylierten Gruppen oder einer beliebigen Kombination davon mit Stickoxid (NO) beladen wird.

4. Medizinische Vorrichtung nach Anspruch 1, 2 oder 3, wobei das Stickoxid eluierende Polymer ausgewählt wird aus der Gruppe, umfassend Aminocellulose, Aminodextran, Chitosan, aminiertes Chitosan, Polyethylenimin, PEI-Cellulose, Polypropylenimin, Polybutylenimin, Polyurethan, Poly(butandiolspermat), Poly(iminocarbonat), Polypeptid, Carboxymethylcellulose (CMC), Polystyrol, Poly(vinylchlorid) und Polydimethylsiloxan oder eine beliebige Kombination davon, und diese genannten Polymere auf eine inerte Hauptkette, wie z.B. eine Polysaccharidhauptkette oder eine zellulosische Hauptkette gepfropft sind.

5. Medizinische Vorrichtung nach Anspruch 1, 2, 3 oder 4, wobei der Protonenspender ausgewählt wird aus der Gruppe, umfassend Wasser, Blut, Lymphe, Galle, Methanol, Ethanol, Propanole, Buthanole, Pentanole, Hexanole, Phenole, Naphtole, Polyole, Phosphate, Succinate, Carbonate, Azetate, Formate, Propionate, Butyrate, Fettsäuren und Aminosäuren oder beliebige Kombinationen davon.

6. Medizinische Vorrichtung nach Anspruch 1, wobei der mikroverkapselte Protonenspender in Mikrokapseln aus Formaldehyd und Gelatine mikroverkapselt ist.

7. Medizinische Vorrichtung nach Anspruch 1, wobei die Vorrichtung eine Folie umfasst, die den in den Mikrokapseln mikroverkapselten Protonenspender umfasst, wobei das NO eluierende Polymer auf diese Folie aufgesponnen ist.

8. Medizinische Vorrichtung nach Anspruch 1, wobei die Vorrichtung das NO eluierende Polymer umfasst, gemischt mit den Mikrokapseln, die den Protonenspender enthalten.

9. Medizinische Vorrichtung nach Anspruch 8, wobei das NO eluierende Polymer, das ausgelegt ist, um NO zu eluieren, in Form von Fasern, Nanoteilchen und/oder Mikrokügelchen bereitgestellt ist.

10. Medizinische Vorrichtung nach Anspruch 1, wobei das NO eluierende Polymer in Form einer Folie, einer Hülle oder eines Klebebands bereitgestellt ist, das auf die Folie, die Hülle oder das Klebeband aufgebracht wird, das den in den Mikrokapseln mikroverkapselten Protonenspender umfasst.

11. Medizinische Vorrichtung nach Anspruch 1, wobei das NO eluierende Polymer ein sekundäres Amin in einer Hauptkette oder ein sekundäres Amin als Pendant umfasst.

12. Medizinische Vorrichtung nach Anspruch 11, wobei sich ein positiver Ligand auf einem dem sekundären Amin benachbarten Kohlenstoffatom befindet.

13. Medizinische Vorrichtung nach Anspruch 1, umfassend ein Absorptionsmittel.

14. Medizinische Vorrichtung nach Anspruch 13, wobei das Absorptionsmittel ausgewählt wird aus der Gruppe, umfassend Polyacrylat, Polyethylenoxid, Carboxymethylcellulose (CMC), microcrystalline Cellulose, Baumwolle oder Stärke oder eine beliebige Kombination davon.

15. Medizinische Vorrichtung nach Anspruch 1 oder 13, umfassend ein Kation zum Stabilisieren des Stickoxid eluierenden Polymers.

16. Medizinische Vorrichtung nach Anspruch 15, wobei das Kation ausgewählt wird aus der Gruppe, umfassend Na⁺, K⁺, Li⁺, Be²⁺, Ca²⁺, Mg²⁺, Ba²⁺ und/oder Sr²⁺ oder eine beliebige Kombination davon.

17. Medizinische Vorrichtung nach Anspruch 10, wobei die Anlagerung mit Leim durchgeführt wird.

18. Medizinische Vorrichtung nach Anspruch 17, wobei der Leim in einem Muster aufgebracht wird, das es ermöglicht, dass der Protonenspender im Innern der Mikrokapseln nach dem Zerbrechen der Mikrokapseln mit dem NO eluierenden Polymer in Kontakt kommt.

19. Medizinische Vorrichtung nach Anspruch 1, wobei die Vorrichtung mit einem Aktivierungsindikator versehen ist.

20. Medizinische Vorrichtung nach Anspruch 19, wobei der Aktivierungsindikator ein Farb-, Duft- und/oder Tonindikator ist.

21. Medizinische Vorrichtung nach Anspruch 1, wobei eine Seite der Vorrichtung eine geringe Durchlässigkeit oder im Wesentlichen keine Durchlässigkeit für Stickoxid aufweist.

22. Medizinische Vorrichtung nach Anspruch 21, wobei die Vorrichtung auf einer ersten Seite der Vorrichtung mit einer Membran versehen ist, die Stickoxid-durchlässig ist, und einer anderen Membran auf einer zweiten Seite der Vorrichtung, die eine geringe Durchlässigkeit oder im Wesentlichen keine Durchlässigkeit für Stickoxid aufweist.

23. Herstellungsverfahren für eine medizinische Vorrichtung nach Anspruch 1, umfassend:
Auswählen einer Vielzahl von Stickoxid (NO) eluierenden Polymerteilchen, einschließlich Nanofasern, Nanoteilchen oder Mikrokügelchen,
Mikroverkapseln eines Protonenspenders, um Mikrokapseln zu bilden, die den Protonenspender enthalten,
Aufbringen der Mikrokapseln auf das Stickoxid (NO) eluierende Polymer, um die Vorrichtung zu bilden.

24. Herstellungsverfahren nach Anspruch 23, ferner umfassend:
Auswählen des Stickoxid (NO) eluierenden Polymers, so dass es ausgelegt ist, um eine therapeutische Dosis von Stickoxid (NO) zu eluieren,
Auswählen eines Trägermaterials, wobei das Trägermaterial ausgelegt ist, um die Elution der therapeutischen Dosis des Stickoxids (NO) zu regulieren und zu steuern,
Einschließen des NO eluierenden Polymers mit dem Trägermaterial in ein Stickoxid (NO) eluierendes Material, damit das Trägermaterial bei Anwendung der Vorrichtung die Elution der therapeutischen Dosis des Stickoxids (NO) reguliert und steuert, und
Bringen des Stickoxid eluierenden Materials in eine geeignete Form oder als Beschichtung auf einen Träger, um zumindest einen Teil der Vorrichtung zu bilden, so dass die Vorrichtung ausgelegt ist, eine therapeutische Zielstelle für das Stickoxid freizulegen, wenn das NO eluierende Polymer bei Anwendung der Vorrichtung Stickoxid (NO) eluiert.

25. Herstellungsverfahren nach Anspruch 24, wobei das Auswählen des Stickoxid (NO) eluierenden Polymers das Auswählen einer Vielzahl von Stickoxid (NO) eluierenden Polymerteilchen, vorzugsweise Nanofasern, Nanoteilchen oder Mikrokügelchen, umfasst.

26. Herstellungsverfahren nach Anspruch 24 oder 23, wobei das Einschließen des NO eluierenden Polymers mit dem Trägermaterial das Integrieren des NO eluierenden Polymers in das Trägermaterial, das Verspinnen des NO eluierenden Polymers zusammen mit dem Trägermaterial oder das Spinnen des NO eluierenden Polymers über dem Trägermaterial umfasst, um die Stickoxid eluierenden Eigenschaften der Vorrichtung vorab zu definieren.

27. Herstellungsverfahren nach Anspruch 23, ferner umfassend das Mikroverkapseln des Protonenspenders in den Mikrokapseln vor dem Aufbringen des Stickoxid (NO) eluierenden Polymers.

28. Herstellungsverfahren nach Anspruch 23, wobei das Aufbringen gemustertes Aufleimen oder Spinnen des NO eluierenden Polymers auf die Mikrokapseln umfasst.

29. Herstellungsverfahren nach Anspruch 23, umfassend das Ausbilden der Mikrokapseln als eine erste Folie, erstes Klebeband oder erste Hülle,
Ausbilden einer zweiten Folie, eines zweiten Klebebands oder einer zweiten Hülle, umfassend das NO eluierende Polymer, und
Leimen der ersten Folie, des ersten Klebebands oder ersten Hülle auf die Mikrokapseln der zweiten Folie, des zweiten Klebebands oder der zweiten Hülle, die das NO eluierende Polymer umfasst.

30. Herstellungsverfahren nach Anspruch 29, wobei das Leimen ein gemustertes Leimen umfasst, so dass ein Muster erhalten wird, das leimfreie Stellen aufweist.

31. Herstellungsverfahren nach Anspruch 23, umfassend das Ausbilden der Mikrokapseln als eine erste Folie, ein erstes Klebeband oder eine erste Hülle, und direktes Spinnen eines Materials, welches das NO eluierende Polymer umfasst, auf die Folie, das Klebeband oder die Hülle aus Mikrokapseln, die den Protonenspender enthalten.

32. Herstellungsverfahren nach Anspruch 23, umfassend das Bereitstellen eines Aktivierungsindikators, der dafür ausgelegt ist, anzuzeigen, wenn die Mikrokapseln zerbrochen sind, damit das NO eluierende Polymer dem Protonenspender ausgesetzt wird, um NO zu eluieren.

33. Herstellungsverfahren nach Anspruch 32, wobei das Bereitstellen eines Aktivierungsindikators das Bereitstellen eines Färbemittels im Innern der Mikrokapseln umfasst.

34. Herstellungsverfahren nach Anspruch 32, wobei das Bereitstellen eines Aktivierungsindikators das Auswählen eines Materials für die Mikrokapseln oder das Auswählen einer Wanddicke der Mikrokapseln umfasst, die einen Ton erzeugen, wenn die Mikrokapseln zerbrechen.

35. Herstellungsverfahren nach Anspruch 32, wobei das Bereitstellen eines Aktivierungsindikators das Beimischen eines Duftstoffes in die Mikrokapseln umfasst.

36. Herstellungsverfahren nach Anspruch 32, wobei das Bereitstellen eines Aktivierungsindikators das Bereitstellen einer Substanz umfasst, welche die Farbe ändert, wenn sie mit dem Protonenspender in Kontakt kommt.

37. Verfahren zum Aktivieren der Stickoxid (NO)-Elution aus der medizinischen Vorrichtung nach Anspruch 1, wobei die Vorrichtung ein NO eluierendes Polymer umfasst, das ausgelegt ist, um bei Kontakt mit einem Protonenspender Stickoxid (NO) daraus zu eluieren, umfassend
Anordnen des NO eluierenden Polymers in der Nähe der Mikrokapseln, die den Protonenspender enthalten, und
Freisetzen des Protonenspenders durch Zerbrechen der Mikrokapseln zum Kontaktieren des NO eluierenden Polymers mit dem Protonenspender.

38. Verfahren nach Anspruch 37, wobei das Zerbrechen mithilfe von Druck, Scherspannung oder Wärme erfolgt.

39. Stickoxid (NO) eluierendes Polymer zur Verwendung in einer medizinischen Vorrichtung oder einem medizinischen System zur Behandlung eines Organs im Körper eines Lebewesens, z.B. eines Menschen,
wobei die medizinische Vorrichtung das Stickoxid (NO) eluierende Polymer umfasst, das dafür ausgelegt ist, eine therapeutische Dosis eines Stickoxids (NO) bei Verwendung der Vorrichtung für die Behandlung zu eluieren, und Mikrokapseln, die einen Protonenspender mit einer Flüssigkeit enthalten, welche Wasser oder eine Wasser enthaltende Flüssigkeit einschließt, und angeordnet ist, zu zerbrechen, um den Protonenspender mit der Flüssigkeit mit dem NO eluierenden Polymer in Kontakt zu bringen, und angeordnet, um das Organ dem Stickoxid auszusetzen, wenn das Polymer bei Verwendung Stickoxid (NO) durch Eluieren der therapeutischen Dosis an Stickoxid aus dem Stickoxid eluierenden Polymer an das Organ eluiert.

## Revendications

1. Dispositif médical, comprenant un polymère d'élution d'oxyde nitrique (NO) configuré pour une élution d'oxyde nitrique (NO) à partir de celui-ci lors d'un contact entre un donneur de protons et ledit polymère d'élution d'oxyde nitrique (NO),
où ledit dispositif est conçu pour être appliqué sur une zone cible sur laquelle l'exposition de l'oxyde nitrique (NO) est souhaitée, et où ledit dispositif est choisi à partir d'un timbre, d'une pommade, d'une bande, d'une chaussette, d'une enveloppe, ou d'une feuille,
**caractérisé en ce que**
ledit dispositif est doté d'un donneur de protons configuré pour ledit contact, et qui est micro-encapsulé dans des microcapsules et où
lesdites microcapsules, dans lesquelles ledit donneur de protons est contenu, sont agencées pour libérer au moins une partie dudit donneur de protons après rupture desdites microcapsules, et
lesdites microcapsules sont agencées de telle sorte que ledit donneur de protons, lorsqu'il est libéré après ladite rupture, entre en contact au moins partiellement avec ledit polymère d'élution d'oxyde nitrique (NO) de telle sorte que l'élution d'oxyde nitrique (NO) à partir dudit polymère d'élution d'oxyde nitrique (NO)est initiée, grâce à quoi ladite élution d'oxyde nitrique (NO) à partir dudit polymère d'élution d'oxyde nitrique (NO) en cours d'utilisation dudit dispositif est prévue sur ladite zone cible.

2. Dispositif médical selon la revendication 1, dans lequel ledit polymère d'élution d'oxyde nitrique (NO) comprend des groupements de diazéniumdiolate, des groupements S-nitrosylés, et des groupements O-nitrosylés, ou une combinaison quelconque de ceux-ci.

3. Dispositif médical selon la revendication 1 ou 2, dans lequel ledit polymère d'élution d'oxyde nitrique (NO) est de la L-PEI (polyéthylèneimine linéaire), chargée avec de l'oxyde nitrique (NO) par l'intermédiaire desdits groupements de diazéniumdiolate, desdits groupements S-nitrosylés, ou desdits groupements O-nitrosylés, ou d'une combinaison quelconque de ceux-ci.

4. Dispositif médical selon la revendication 1, 2 ou 3, dans lequel ledit polymère d'élution d'oxyde nitrique est sélectionné parmi le groupe comprenant l'amino-cellulose, des amino-dextranes, le chitosane, le chitosane aminé, la polyéthylèneimine, la PEI-cellulose, la polypropylèneimine, la polybutylèneimine, le polyuréthane, le poly(spermate de butanediol), le poly(iminocarbonate), un polypeptide, la carboxyméthylcellulose (CMC), le polystyrène, le poly(chlorure de vinyle) et le polydiméthylsiloxane, ou des combinaisons quelconques de ceux-ci, et ces polymères mentionnés greffés à un squelette inerte, tel qu'un squelette de polysaccharide ou un squelette cellulosique.

5. Dispositif médical selon la revendication 1, 2, 3 ou 4, dans lequel ledit donneur de protons est sélectionné parmi le groupe comprenant l'eau, le sang, la lymphe, la bile, le méthanol, l'éthanol, les propanols, les butanols, les pentanols, les hexanols, les phénols, les naphtols, les polyols, les phosphates, les succinates, les carbonates, les acétates, les formates, les propionates, les butyrates, les acides gras, et les acides animés, ou des combinaisons quelconques de ceux-ci.

6. Dispositif médical selon la revendication 1, dans lequel ledit donneur de protons micro-encapsulé est micro-encapsulé dans des microcapsules de formaldéhyde et de gélatine.

7. Dispositif médical selon la revendication 1, dans lequel ledit dispositif comprend un film comprenant ledit donneur de protons micro-encapsulé dans lesdites microcapsules, où ledit polymère d'élution de NO est filé sur ledit film.

8. Dispositif médical selon la revendication 1, dans lequel ledit dispositif comprend ledit polymère d'élution de NO mélangé avec lesdites microcapsules contenant ledit donneur de protons.

9. Dispositif médical selon la revendication 8, dans lequel ledit polymère d'élution de NO, configuré pour éluer du NO, est fourni sous la forme de fibres, de nanoparticules et/ou de microsphères.

10. Dispositif médical selon la revendication 1, dans lequel ledit polymère d'élution de NO est prévu sous la forme d'un film, d'un fourreau ou d'une bande, qui est fixé sur un film, un fourreau ou une bande comprenant ledit donneur de protons, micro-encapuslé dans lesdites microcapsules.

11. Dispositif médical selon la revendication 1, dans lequel ledit polymère d'élution de NO comprend une amine secondaire dans un squelette ou une amine secondaire en tant que ramification.

12. Dispositif médical selon la revendication 11, dans lequel un ligand positif est situé sur un atome de carbone voisin pour l'amine secondaire.

13. Dispositif médical selon la revendication 1, comprenant un agent absorbant.

14. Dispositif médical selon la revendication 13, dans lequel ledit agent absorbant est sélectionné parmi le groupe comprenant le polyacrylate, l'oxyde de polyéthylène, la carboxyméthylcellulose (CMC), la cellulose microcristalline, le coton, ou l'amidon, ou des combinaisons quelconques de ceux-ci.

15. Dispositif médical selon la revendication 1 ou 13, comprenant un cation destiné à stabiliser le polymère d'élution d'oxyde nitrique.

16. Dispositif médical selon la revendication 15, dans lequel ledit cation est sélectionné parmi le groupe comprenant Na⁺, K⁺, Ni⁺, Be²⁺, Ca²⁺, Mg²⁺, Ba²⁺ et/ou Sr²⁺, ou des combinaisons quelconques de ceux-ci.

17. Dispositif médical selon la revendication 10, dans lequel ladite fixation est réalisée avec de la colle.

18. Dispositif médical selon la revendication 17, dans lequel ladite colle est appliquée suivant un motif permettant au donneur de protons à l'intérieur desdites microcapsules d'entrer en contact avec ledit polymère d'élution de NO après rupture desdites microcapsules.

19. Dispositif médical selon la revendication 1, où ledit dispositif est doté d'un indicateur d'activation.

20. Dispositif médical selon la revendication 19, dans lequel ledit indicateur d'activation se présente sous la forme d'un indicateur coloré, odorant, et/ou sonore.

21. Dispositif médical selon la revendication 1, où un côté du dispositif présente une faible perméabilité, ou pratiquement aucune perméabilité, à l'oxyde nitrique.

22. Dispositif médical selon la revendication 21, où le dispositif est doté d'une première membrane, qui est perméable à l'oxyde nitrique, sur un premier côté du dispositif, et d'une autre membrane, qui présente une faible perméabilité ou pratiquement aucune perméabilité à l'oxyde nitrique, sur un deuxième côté dudit dispositif.

23. Procédé de fabrication d'un dispositif médical selon la revendication 1, comprenant :
la sélection d'une pluralité de particules polymères d'élution d'oxyde nitrique (NO), comprenant des nanofibres, des nanoparticules ou des microsphères,
la micro-encapsulation d'un donneur de protons pour former des microcapsules contenant ledit donneur de protons,
l'application desdites microcapsules sur ledit polymère d'élution d'oxyde nitrique (NO) pour former ledit dispositif.

24. Procédé de fabrication selon la revendication 23, comprenant en outre
la sélection dudit polymère d'élution d'oxyde nitrique (NO) de telle sorte qu'il est configuré pour éluer un dosage thérapeutique d'oxyde nitrique (NO),
la sélection d'un matériau vecteur, lequel matériau vecteur est configuré pour réguler et contrôler l'élution dudit dosage thérapeutique d'oxyde nitrique (NO),
l'incorporation du polymère d'élution de NO audit matériau vecteur dans un matériau d'élution d'oxyde nitrique (NO), de telle sorte que ledit matériau vecteur, au cours de l'utilisation dudit dispositif, régule et contrôle l'élution dudit dosage thérapeutique d'oxyde nitrique (NO), et
le déploiement dudit matériau d'élution d'oxyde nitrique sous une forme appropriée, ou en tant que revêtement sur un support, pour former au moins une partie dudit dispositif, de telle sorte que ledit dispositif est configuré pour exposer un site cible thérapeutique audit oxyde nitrique lorsque ledit polymère d'élution de NO en cours d'utilisation élue l'oxyde nitrique (NO).

25. Procédé la fabrication selon la revendication 24, dans lequel ladite sélection dudit polymère d'élution d'oxyde nitrique (NO) comprend la sélection d'une pluralité de particules polymères d'élution d'oxyde nitrique (NO), de préférence des nanofibres, des nanoparticules ou des microsphères.

26. Procédé de fabrication selon la revendication 24 ou 23, dans lequel ladite incorporation dudit polymère d'élution de NO audit matériau vecteur comprend l'intégration dudit polymère d'élution de NO dans ledit matériau vecteur, le filage dudit polymère d'élution de NO avec ledit matériau vecteur, ou le filage dudit polymère d'élution de NO sur la partie supérieure dudit matériau vecteur, de manière à prédéfinir les caractéristiques d'élution d'oxyde nitrique dudit dispositif.

27. Procédé de fabrication selon la revendication 23, comprenant en outre la micro-encapsulation dudit donneur de protons dans lesdites microcapsules, avant le déploiement dudit polymère d'élution d'oxyde nitrique (NO).

28. Procédé de fabrication selon la revendication 23, dans lequel ladite application comprend l'encollage en motif, ou le filage du polymère d'élution de NO sur lesdites microcapsules.

29. Procédé de fabrication selon la revendication 23, comprenant le fait de former les microcapsules dans un premier film, bande ou fourreau,
le fait de former un deuxième film, bande ou fourreau comprenant ledit polymère d'élution de NO, et
l'encollage du premier film, bande ou fourreau de microcapsules sur ledit deuxième film, bande ou fourreau comprenant ledit polymère d'élution de NO.

30. Procédé de fabrication selon la revendication 29, dans lequel ledit encollage comprend l'encollage en motif, de telle sorte qu'un motif comprenant des espaces exempts de colle est obtenu.

31. Procédé de fabrication selon la revendication 23, comprenant le fait de former les microcapsules en un premier film, bande ou fourreau, et le filage de manière directe d'un matériau comprenant le polymère d'élution de NO sur le film, bande ou fourreau de microcapsules, contenant un donneur de protons.

32. Procédé de fabrication selon la revendication 23, comprenant la fourniture d'un indicateur d'activation configuré pour indiquer lorsque les microcapsules sont rompues de telle sorte que le polymère d'élution de NO est soumis audit donneur de protons pour éluer le NO.

33. Procédé de fabrication selon la revendication 32, dans lequel ladite fourniture d'un indicateur d'activation comprend la fourniture d'un agent de coloration à l'intérieur des microcapsules.

34. Procédé de fabrication selon la revendication 32, dans lequel ladite fourniture d'un indicateur d'activation comprend la sélection d'un matériau pour les microcapsules, ou le choix de l'épaisseur de paroi desdites microcapsules, qui crée un son lorsque les microcapsules se rompent.

35. Procédé de fabrication selon la revendication 32, dans lequel ladite fourniture d'un indicateur d'activation comprend l'ajout au mélange d'un matériau odorant dans les microcapsules.

36. Procédé de fabrication selon la revendication 32, dans lequel ladite fourniture d'un indicateur d'activation comprend la fourniture d'une substance qui change de couleur lorsqu'elle entre en contact avec le donneur de protons.

37. Procédé d'activation d'une élution d'oxyde nitrique (NO) à partir d'un dispositif médical selon la revendication 1, ledit dispositif comprenant un polymère d'élution de NO configuré pour éluer l'oxyde nitrique (NO) à partir de celui-ci lors d'un contact avec un donneur de protons, comprenant
l'agencement dudit polymère d'élution de NO à proximité des microcapsules contenant ledit donneur de protons, et
la libération dudit donneur de protons en rompant lesdites microcapsules pour un contact dudit polymère d'élution de NO avec ledit donneur de protons.

38. Procédé selon la revendication 37, dans lequel ladite rupture est réalisée grâce à une pression, un déchirement ou la chaleur.

39. Polymère d'élution d'oxyde nitrique (NO) destiné à être utilisé dans un dispositif médical ou un système pour traiter un organe d'un corps d'un animal, comme un être humain,
où le dispositif médical comprend le polymère d'élution d'oxyde nitrique (NO) qui est configuré pour éluer un dosage thérapeutique d'oxyde nitrique (NO) lorsqu'il est utilisé pour ledit traitement et des microcapsules, contenant un liquide contenant un donneur de protons, y compris l'eau ou un liquide contenant de l'eau, agencées pour se rompre afin de mettre ledit liquide contenant un donneur de protons en contact avec ledit polymère d'élution de NO, et agencées pour exposer ledit organe audit oxyde nitrique lorsque ledit polymère en cours d'utilisation élue l'oxyde nitrique (NO) par l'élution dudit dosage thérapeutique d'oxyde nitrique à partir dudit polymère d'élution d'oxyde nitrique sur ledit organe.
